# EUROPÄISCHE PATENTSCHRIFT

(11) **EP 1 119 351 B1**
(45) Veröffentlichungstag und Bekanntmachung des Hinweises auf die Patenterteilung: **02.05.2003**
(21) Anmeldenummer: 99969932.5
(22) Anmeldetag: 23.09.1999
(51) Int. Cl.: A61K 31/05, A61K 31/075, A61K 31/235, A61K 31/415, A61P 9/10, A61P 17/16, A61P 19/10, A61P 27/02, A61P 35/00

(54) **VERWENDUNG VON KATECHOLDERIVATEN ALS PROTEINASEINHIBITOREN**
USE OF CATECHOL DERIVATIVES AS PROTEINASE INHIBITORS
UTILISATION DE DERIVES DE CATECHINE COMME INHIBITEURS DE PROTEINASE

(30) Priorität: 02.10.1998 DE 19845372
(43) Veröffentlichungstag der Anmeldung: 01.08.2001
(73) Patentinhaber: Grünenthal GmbH, 52078 Aachen (DE)
(72) Erfinder: SEIPP, Ulrich, D-52076 Aachen (DE); ZIMMER, Oswald, D-52146 Würselen (DE); STRASSBURGER, Wolfgang, D-52146 Würselen (DE); SCHNEIDER, Johannes, D-52223 Stolberg (DE); WNENDT, Stephan, D-52076 Aachen (DE); ULBRICH, Norbert, D-14469 Potsdam (DE); HECKER-KIA, Heidi, D-12203 Berlin (DE); ZIMMERMANN, Bernd, D-14195 Berlin (DE)
(86) Internationale Anmeldenummer: EP9907068
(87) Internationale Veröffentlichungsnummer: WO00019989

(56) Entgegenhaltungen:
- WO-A-82/03729
- WO-A-88/01509
- WO-A-95/00129
- WO-A-96/31206
- WO-A-97/11692
- DE-A- 3 518 655
- CHEMICAL ABSTRACTS, vol. 131, no. 8, 23. August 1999 (1999-08-23) Columbus, Ohio, US; abstract no. 97593, SAKAI, YUKIMICHI ET AL: "Magnolol and honokiol from Magnolia bark as cancer metastasis inhibitors and collagenase inhibitors" XP002140193 & JP 11 209276 A (GIFU PREFECTURE, JAPAN) 3. August 1999 (1999-08-03)
- SCHADE, ULRICH F. ET AL: "Lipoxygenase inhibitors suppress formation of tumor necrosis factor in vitro and in vivo" BIOCHEM. BIOPHYS. RES. COMMUN. (1989), 159(2), 748-54 , XP000914523

## Beschreibung

Die Erfindung betrifft die Verwendung ausgewählter Katecholderivate zur Herstellung eines Medikaments zur Behandlung von Erkrankungen, an deren Pathogenese Elastase und/oder Metalloproteinasen beteiligt sind.

Metalloproteinasen und die Serinproteinase Elastase spielen eine zentrale Rolle bei der Entstehung und dem Verlauf entzündlicher Erkrankungen im menschlichen Körper wie der rheumatoiden Arthritis, der Parodontitis, der Reaktion der Haut auf UV-Strahlung sowie primär nicht entzündlicher Erkrankungen wie der Entstehung arteriosklerotischer Plaques, der Osteoporose und der Arthrose. Die Funktion der Metalloproteinasen im Verlauf dieser Vorgänge besteht einerseits im Abbau von Matrixgewebe, andererseits in der Aktivierung von proinflammatorischen Vorläuferproteinen.

Die bekannten Proteinaseninhibitoren sind in der -Regel komplexe Moleküle, die für die Hemmung von Metalloproteinasen verwendet werden. Hierzu zählen neben Tetrazyklinderivaten größtenteils die Peptidomimetica (Beckett et al., Drug Discovery Today, (1996), S. 16 - 26). Damit ergibt sich das Problem begrenzter oraler Verfügbarkeit für die Peptidomimetica, da diese Substanzen von relativ unspezifischen Peptidasen im Magen-Darm-Trakt verdaut werden. Die Verbindungen der allgemeinen Formel I sind beispielsweise aus EP-A-0 202 529 und WO 96/31206 als Lipoxygenaseinhibitoren und Antihystaminika und aus WO 82/03729 als Hilfsmittel zur Modifikation von Elektroden bekannt. WO 97/11692 offenbart die Verwendung von Tyrosin Kinase-Inhibitoren zur Behandlung von Osteoarthritis.

Die Aufgabe der vorliegenden Erfindung bestand darin, Verbindungen zur Verfügung zu stellen, die als Proteinasinhibitoren zur Behandlung von Erkrankungen, an deren Pathogenese Metalloproteinasen und/oder Elastase beteiligt sind, verwendet werden.

Es wurde nun gefunden, daß die an die Verbindungen gestellten Anforderungen von ausgewählten Katecholderivaten erfüllt wurden. Die Verbindungen zeichnen sich durch eine ausgeprägte Hemmung der Metalloproteinase- und/oder Elastase-Aktivität aus.

Gegenstand der Erfindung sind dementsprechend ausgewählte Katecholverbindungen, die unter die allgemeine Formel I fallen 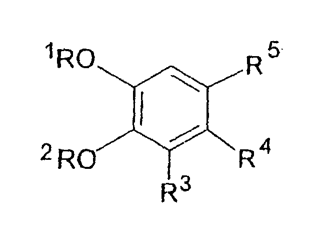 worin
- R¹: H, Aryl-Heterocyclyl, C₁₋₁₆-Alkyl, Aryl, CHO, CON(CH₃)₂, COCH₃, CO-tert.Butyl, Heterocyclyl oder C₂₋₁₆-Alkenyl;
- R²: H, Aryl-Heterocyclyl, C₁₋₁₆-Alkyl, Aryl, CHO, CON(CH₃)₂, COCH₃, CO-tert.Butyl, Heterocyclyl oder C₂₋₁₆-Alkenyl;
- R³: H,OH,C₂₋₁₆-Alkoxy oder C₂₋₆-Alkenyl-COO-C₁₋₆-Alkyl;
- R⁴: Aryl, -CH=CH-Aryl, H, unsubstituiertes und/oder mit OH, NH₂ oder Halogen substituiertes C₂₋₁₆-Mono- oder Dialkinyl, Heterocyclyl, -C(O)-Heterocyclyl, C₁₋₈-Alkyl oder R³ und R⁴ zusammen mit den C-Atomen des aromatischen Rings einen 4 bis 6 gliedrigen gesättigten oder teilweise ungesättigten cyclischen Kohlenwasserstoffring bilden und
- R⁵: Aryl, -CH=CH-Aryl, H, unsubstituiertes und/oder mit OH, NH₂ oder Halogen substituiertes C₂₋₁₆-Mono- oder Dialkinyl, Heterocyclyl, -C(O)-Heterocyclyl, C₁₋₈-Alkyl oder R⁴ und R⁵ zusammen mit den C-Atomen des aromatischen Rings einen 4 bis 6 gliedrigen gesättigten oder teilweise ungesättigten cyclischen Kohlenwasserstoffring bilden
in Form der Basen oder pharmazeutisch verwendbarer Salze.

Der Ausdruck "C₁₋₁₆-Alkyl" bedeutet geradkettige oder verzweigte Kohlenwasserstoffe mit 1-16 Kohlenstoffatomen. Beispielhaft seien Methyl, Ethyl, Propyl, n-Butyl, sek.-Butyl, n-Pentyl, neo-Pentyl, n-Hexyl, n-Heptyl, 2,3,4-Trimethyl-heptyl, 2,2,3, 4,5,5,6-Heptamethyl-octyl, n-Nonyl, 2, 6-Diethyl-3,4,5-Trimethyl-Decanyl, n-Undecanyl, n-Dodecanyl und 3-Ethyl-4-methyl-dodecanyl genannt.

Der Ausdruck "C₁₋₁₆-Alkoxy" bedeutet geradkettige oder verzweigte Kohlenwasserstoffe mit 1-16 Kohlenstoffatomen, wie oben definiert, die über das Sauerstoffatom gebunden sind.

Der Ausdruck "C₂₋₆-Alkenyl" bzw. "C₂₋₁₆-Alkenyl" bedeutet geradkettige oder verzweigte Kohlenwasserstoffe mit 2 bis 6 bzw. 2 bis 16 Kohlenstoffatomen, wie oben definiert, die zusätzlich eine freie Doppelbindung innerhalb der Kohlenstoffkette besitzen.

Der Ausdruck "C₂₋₁₆-Mono- oder Dialkinyl" bedeutet geradkettige oder verzweigte Kohlenwasserstoffe mit 2 bis 16 Kohlenstoffatomen, wie oben definiert, die zusätzlich eine oder zwei freie Dreifachbindungen innerhalb der Kohlenstoffkette besitzen. Zusätzlich kann der Kohlenwasserstoffrest Substituenten aus der Gruppe von OH, NH₂ und/oder Halogen enthalten.

Der Ausdruck "Aryl" bedeutet unsubstituierte oder ein- oder mehrfach mit OH, F, Cl, CF₃, C₁₋₁₆-Alkyl, C₁₋₁₆-Alkoxy, C₃₋₇-Cycloalkyl, C₂₋₁₆-Alkenyl, Heterocyclyl oder Phenyl substituierte Phenyle oder Naphthyle. Gegebenenfalls können die Heterocyclyl- oder Phenylreste ankondensiert sein.

Der Ausdruck "Heterocyclyl" bedeutet 5- oder 6-gliedrige gesättigte oder ungesättigte, unsubstituierte oder ein- oder mehrfach mit OH, F, Cl, CF₃, C₁₋₁₆-Alkyl, C₁₋₁₆-Alkoxy, C₂₋₁₆-Alkenyle, mono- oder Di-C₂₋₁₆-Alkinylen, C₂₋₁₆- gesättigte oder ungesättigte Carbonsäuren oder Carbonsäureester mit 2 bis 16 Kohlenstoffatomen für den Kohlenwasserstoffanteil der Carbonsäure und 1 bis 6 Kohlenstoffatome für die Kohlenwasserstoffanteil im Ester, Heterocyclyl oder Phenyl substituierte, gegebenenfalls mit einem ankondensierten Arylsystem versehene, heterocyclische Verbindungen, die 1 oder 2 Heteroatome aus der Gruppe von Stickstoff, Sauerstoff und/oder Schwefel enthalten.

Beispielhaft seien für die gesättigten Heterocyclyle 1,4-Dioxan, Tetrahydrofuran, Pyrrolidin, Oxazolidin und 1,4-Thioxan aufgeführt.

Aus der Gruppe der ungesättigten Heterocyclyle seien beispielhaft Furan, Thiophen, Pyridin, Pyrimidin, Pyrazol, Thiopyran, Pyran, Thiazol, Oxazol, Isoxazol, Pyridazin, Pyrazin, Chinolin, Isochinolin, Phthalazin und Chinazolin aufgeführt.

Unter dem Begriff "Aryl-Heterocyclyl" wird "Aryl" und "Heterocyclyl" wie oben definiert verstanden, die über eine Einfachbindung miteinander verbunden sind.

Beispielhaft sind Verbindungen in denen die Reste R¹ und R² Wasserstoff und die Reste R³ bis R⁵ die vorgenannte Bedeutung besitzen.

Die erfindungsgemäßen Verbindungen sind:
5,6,7-Trihydroxy-3,4-dihydronaphthalin-2-carbonsäure methylester, 4-Nonyloxy-1,2-benzoldiol;
(3,4-Dihydroxy-phenyl)(5-phenyl-2H-pyrazol-3-yl)-methanon;
4-(2-Naphth-2-yl-vinyl)-1,2-benzoldiol; und
4-(11-Hydroxy-undeca-1,9-diynyl)-1,2-benzoldiol.

Die erfindungsgemäßen Verbindungen finden Verwendung zur Herstellung eines Medikaments zur Behandlung von Erkrankungen aus der Gruppe von rheumatoider Arthritis, Parodontitis, arteriosklerotischer Plaques, Osteoporose, Arthrose, Ulzeration der Cornea und Reaktionen der Haut auf UV-Strahlung.

Die erfindungsgemäßen Verbindungen finden speziell Verwendung zur Herstellung eines Medikaments zur Behandlung von rheumatoider Arthritis, Parodontitis, arteriosklerotischer Plaques, Osteoporose, und Arthrose.

Bevorzugt finden die erfindungsgemäßen Katecholderivate Verwendung zur Herstellung eines Medikaments zur Behandlung der Osteoporose, arteriosklerotischer Plaques und der Arthrose.

### Beispiele

Die nachfolgenden Beispiele dienten zum Nachweis der erfindungsgemäßen Aktivität der erfindungsgemäßen Katecholverbindungen an Matrix Metalloproteinasen (MMP' s-) und Elastase.

Hierzu erfolgten Untersuchungen mit unterschiedlichen Matrix-Metalloproteinasen aus der Gruppe von Humanfibroblasten-Kollagenese (MMP-1), Stromelysin-1 (MMP-3), Humanleukozyten-Kollagenese (MMP-8) und humaner Gelatinase B (MMP-9) und an humaner Elastase, die die Hemmung mit den erfindungsgemäßen Proteinaseinhibitoren belegten. Die Untersuchungen wurden in vitro mit gereinigten humanen Enzymen durchgeführt. Als Enzymsubstrate wurden photometrisch bzw. fluorometrisch zu vermessende Substrate eingesetzt. In der nachfolgenden Tabelle 1 wurden die Ergebnisse zusammengefaßt. Durch die Daten wurde belegt, daß die erfindungsgemäßen Verbindungen metalloproteinaseinhibitorische Eigenschaften aufwiesen. Die Hemmung von Elastase wurde ebenfalls nachgewiesen. Die Verbindungen zeigten unterschiedliche Aktivität bei der Hemmung der Elastase.

Folgende Verbindungen wurden in den nachfolgenden Beispielen in der vorgegebenen Reihenfolge verwendet:
Beispiel 1: 5,6,7-Trihydroxy-3, 4-dihydronaphthalin-2-carbonsäuremethylester;
Beispiel 3: 4-Nonyloxy-1,2-benzoldiol;
Beispiel 5: 4-(2-Naphth-2-yl-vinyl)-1,2-benzoldiol;
Beispiel 6: (3,4-Dihydroxy-phenyl)(5-phenyl-2H-pyrazol-3-yl)-methanon;

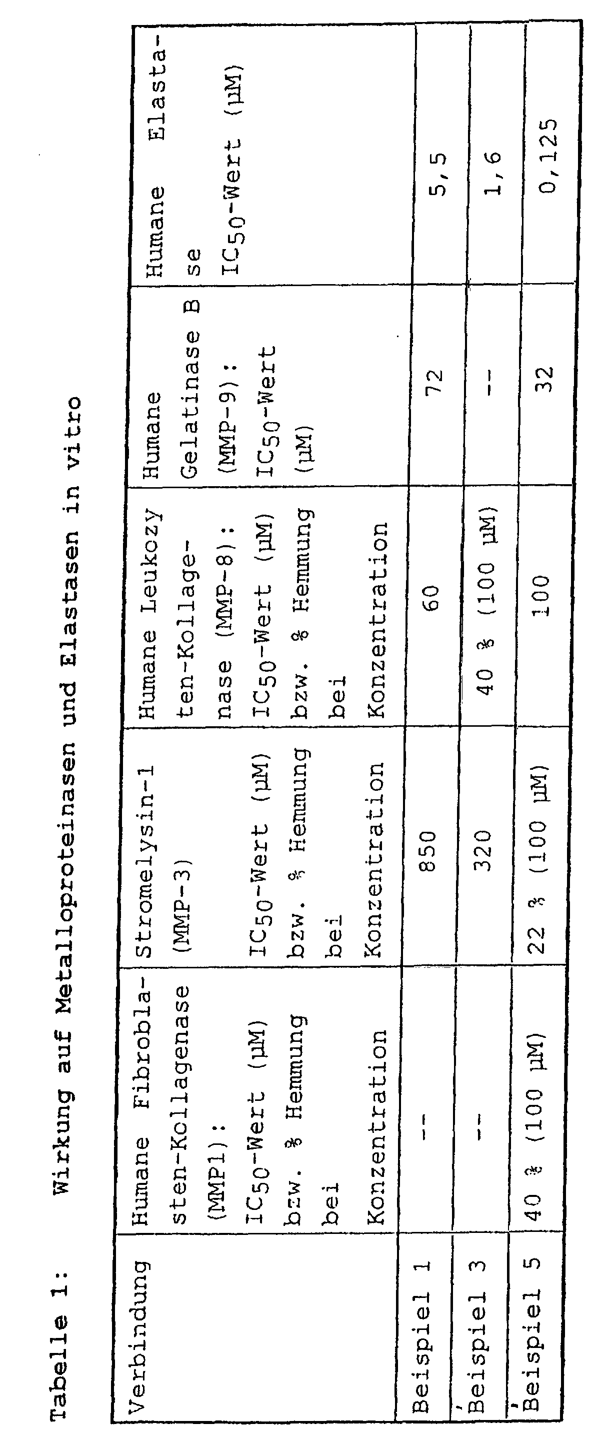

Die erfindungsgemäßen Verbindungen zeigten neben der Hemmung der Metalloproteinasen ebenfalls eine Hemmung bezüglich der Elastase (Tabelle 1).

In einem weiteren in vitro Experiment wurde die Wirksamkeit der Verbindungen bezüglich entzündlicher Erkrankungen in einem Organoidmodell zur Knorpelbildung überprüft. Für dieses Modell wurden mesenchymale Zellen aus den Extremitätenanlagen muriner Embryonen in Zellkultur gebracht. Diese Zellen bildeten in der Zellkultur eine extrazelluläre Matrix, die dem Gelenkknorpel entsprach. Durch Zugabe von β-Glycerophospat wurde die Mineralisation der extrazellulären Matrix induziert. Die Knorpelbildung und die Mineralisation wurden durch Zugabe von bakteriellem Lipopolysaccharid (LPS) als Entzündungsmediator gehemmt. Der Effekt der Verbindungen auf die durch LPS hervorgerufene Störung der Mineralisation wurde überprüft, indem der Calciumgehalt der extrazellulären Matrix flammenfotometrisch nach 14-tägiger Kultivierung gemessen wurde. Der Einfluß auf die Knorpelbildung wurde in der Weise nachgewiesen, daß der Proteoglycan-Gehalt der extrazellulären Matrix mit dem Farbstoff Alcian Blau nach einem Zeitraum von 4 bis 16 Tagen Behandlung überprüft wurde.

**Tabelle 2:**

| **Effekt der in vitro wirksamen Verbindungen im Organoid-Modell** | |
|---|---|
| Verbindung | Chondrozyten: Mineralisation in Gegenwart von LPS; Wirkung bei einer Konzentration von 10 µM |
| Beispiel 1 | + 42 % |
| Beispiel 3 | + 43 % |
| Beispiel 5 | + 81 % |
| Beispiel 6 | + 17 % |

Die Werte geben die prozentuale Steigerung der Mineralisation im Vergleich zur LPS-behandelten Kultur wieder.

Die in Tabelle 2 dargestellten Ergebnisse zeigten, daß die durch LPS induzierten Reduktionen der Matrixmineralisation mittels der erfindungsgemäßen Verbindungen antagonisiert wurden. Als Maßstab für die chondroprotektive Wirkung diente eine gesteigerte Alcian-Blau-Bindung für den Proteoglycan-Gehalt der extrazellulären Matrix. Die Verbindungen stimulierten nicht nur in Gegenwart sondern auch in Abwesenheit von LPS den Erhalt der extrazellulären Matrix. Die Steigerung der Alcian-Blau-Bindung war konzentrationsabhängig. Einige Verbindungen zeigten über den zeitlichen Verlauf, daß sie durch die Hemmung des Abbaus einen massiven chondroprotektiven Effekt induzierten. Dieser Effekt ging mit einer deutlichen Hemmung der Freisetzung von Prostaglandin E₂ (PGE₂) in das Zellkulturmedium des Organoidmodells einher. Erhöhte PGE₂-Konzentrationen wurden auch in der Synovialflüssigkeit arthritischer Patienten nachgewiesen. Prostaglandin E₂ (PGE₂) ist ein bekanntes Produkt des Arachidonsäuremetabolismus aus dem Cyclooxygenaseweg. Dieses Prostaglandin steigert die pronozizeptive Wirkung von Bradykinin, verursacht Vasodilatation und wirkt ödemfördernd.

In einem weiteren Versuch wurden die überraschenden Ergebnisse des in vitro-Beispiels und der im murinen Organoidmodell gewonnenen Befunde am Beispiel der rheumatoiden Arthritis überprüft. Die Katechoderivate wurden in einer Zellkultur humaner Synovial-Fibroblasten getestet. Das für die Untersuchungen eingesetzte Zellmaterial stammte ursprünglich aus arthritischen Kniegelenken.

Die Fibroblastenkulturen wurden mit den unterschiedlichen Stimuli Lipopolysaccharid (LPS), Interleukin-1 (IL 1) und einem Neuropeptid der Substanz P behandelt. Bei dem Lipopolysaccharid und dem Interleukin-1 handelt es sich um proinflammatorische Mediatoren. Die Substanz P ist ein Neurotransmitter der afferenten Neuronen, der an Entzündungsvorgängen und der Schmerzverarbeitung beteiligt ist (N. Otsuka and K. Yoshioka, The Am. Physiol. Soc., Vol. 73, No.2, April 1993, 229-308). Als Meßparameter diente die Freisetzung von Interleukin 6 (IL-6), einem Entzündungsmediator, und die Aktivität der sekretierten Metalloproteinasen. Die Freisetzung von IL-6 wurde durch die erfindungsgemäßen Katecholderivate gehemmt.

Die Matrix-Metalloproteinase-Aktivität im Zellkulturmedium wurde mit einem fluorogenen Substrat (Knight CG et al. (1992), FEBS Lett. 296, 263) gemessen. Die proteolytische Aktivität der Proben, ohne Zugabe von erfindungsgemäßen Katecholderivaten, wurde als 100 % angesehen. Die Zellkulturmedien, die mit Verbindungen mit einer Konzentration von (10⁻⁵ M) behandelt wurden, zeigten abhängig von der in vitro Aktivierung der Pro-Enzyme eine Hemmung der proteolytischen Aktivität.

Nach Inkubation mit Trypsin und Trypsin-Inhibitor (in diesem System wurde hauptsächlich die interstitielle Kollagenase, MMP-1, aktiviert) wurde bei einigen Verbindungen eine Hemmung von 90 % beobachtet, wogegen bei einer Aktivierung mit 4-Aminophenylquecksilberacetat (APMA), welches hauptsächlich die Gelatinase A aktivierte, nur eine geringe Hemmung initiert wurde. Die Stromelysin-1-Aktivität (MMP-3), gemessen mit einem spezifischen fluorogenen Substrat (Nagase et al. (1994), J. Biol. Chem. 269, 20952), wurde im Vergleich zum unbehandelten Kontrollmedium zu 85 % gehemmt.

Die Stimulierung von Synovial-Fibroblasten durch Lipopolysaccharide, IL-1 und dem Neuropeptid Substanz P, führte in Gegenwart von erfindungsgemäßen Katecholderivaten zu einer reduzierten Matrix Metalloproteinase-Aktivität.

Nach Lipopolysaccharid-Stimulierung wurden die 4-Aminophenylquecksilberacetat (APMA), aktivierbaren Matrix Metalloproteinase-Aktivität (MMPs), d. h. in diesem Fall die Gelatinase A, nur schwach gehemmt, wogegen die Trypsin-aktivierbaren Matrix Metalloproteinase-Aktivität (MMPs), d. h. Stromelysin-1 (MMP-3) gehemmt wurden.

Die Hemmwirkung der Verbindungen auf die Matrix Metalloproteinase-Aktivitäten von IL-1-stimulierten Synovial-Fibroblasten konnte ebenfalls nachgewiesen werden.

Die AMPA-aktivierbare Matrix Metalloproteinase-Aktivität, d. h. Gelatinase A, wurde nach Stimulierung mit dem Neuropeptid Substanz P nicht gehemmt, wogegen die Trypsin-aktivierbaren Matrix Metalloproteinasen d. h. hauptsächlich die interstitielle Kollagenase und Stromelysin-1, zu 75 % gehemmt wurden.

## Patentansprüche

1. Verwendung von Katecholderivaten ausgewählt aus der Grupppe:
5,6,7-Trihydroxy-3,4-dihydronaphthalin-2-carbonsäuremethylester;
4-Nonyloxy-1,2-benzoldiol;
(3,4-Dihydroxy-phenyl)(5-phenyl-2H-pyrazol-3-yl)-methanon und
4-(11-Hydroxy-undeca-1,9-diynyl)-1,2-benzoldiol
in Form der Basen oder pharmazeutisch verwendbarer Salze, zur Herstellung eines Medikaments zur Behandlung von Erkrankungen, an deren Pathogenese Elastase und/oder Metalloproteinasen beteiligt sind.

2. Verwendung gemäß Anspruch 1, **dadurch gekennzeichnet, daß** das Medikament zur Behandlung von Erkrankungen aus der Gruppe: rheumatoider Arthritis, Parodontitis, arteriosklerotischer Plaques, Osteoporose, Arthrose, Ulzeration der Cornea und Reaktionen der Haut auf UV-Strahlung, dient.

3. Verwendung gemäß Anspruch 1, **dadurch gekennzeichnet, daß** das Medikament zur Behandlung von Erkrankungen aus der Gruppe: rheumatoider Arthritis, Parodontitis, arteriosklerotischer Plaques, Osteoporose, Arthrose, dient.

4. Verwendung von 4-(2-Naphth-2-yl-vinyl)-1,2-benzoldiol in Form der Basen oder pharmazeutisch verwendbarer Salze, zur Herstellung eines Medikaments zur Behandlung von Erkrankungen, an deren Pathogenese Elastase und/oder Metalloproteinasen beteiligt sind.

5. Verwendung gemäß Anspruch 4, **dadurch gekennzeichnet, daß** das Medikament zur Behandlung von Erkrankungen aus der Gruppe: rheumatoider Arthritis, Parodontitis, Osteoporose, arteriosklerotische Plaques, Arthrose, dient.

## Claims

1. Use of catechol derivatives selected from the group:
5,6,7-trihydroxy-3,4-dihydronaphthalene-2-carboxylic acid methyl ester,
4-nonyloxy-1,2-benzenediol,
(3,4-dihydroxyphenyl) (5-phenyl-2H-pyrazol-3-yl)-methanone, and
4-(11-hydroxyundeca-1,9-diynyl)-1,2-benzenediol,
in the form of the bases or pharmaceutically usable salts, for the production of a medicament for the treatment of diseases in the pathogenesis of which elastase and/or metalloproteinases are involved.

2. Use according to claim 1, **characterised in that** the medicament serves for the treatment of diseases from the group comprising: rheumatoid arthritis, periodontitis, arteriosclerotic plaques, osteoporosis, arthrosis, ulceration of the cornea, and reactions of the skin to UV radiation.

3. Use according to claim 1, **characterised in that** the medicament serves for the treatment of diseases from the group comprising: rheumatoid arthritis, periodontitis, arteriosclerotic plaques, osteoporosis and arthrosis.

4. Use of 4-(2-naphth-2-yl-vinyl)-1,2-benzenediol in the form of the bases or pharmaceutically usable salts, for the production of a medicament for the treatment of diseases in the pathogenesis of which elastase and/or metalloproteinases are involved.

5. Use according to claim 4, **characterised in that** the medicament serves for the treatment of diseases from the group comprising: rheumatoid arthritis, periodontitis, osteoporosis, arteriosclerotic plaques and arthrosis.

## Revendications

1. Utilisation de dérivés de catéchol choisis dans le groupe :
ester méthylique d'acide 5,6,7-trihydroxy-3,4-dihydronaphtalène-2-carboxylique ;
4-nonyloxy-1,2-benzènediol ;
(3,4-dihydroxy-phényl)(5-phényl-2H-pyrazole-3-yl)-méthanone et
4-(11-hydroxy-undeca-1,9-diynyl)-1,2-benzènediol
sous forme des bases ou de sels acceptables du point de vue pharmaceutique, pour la préparation d'un médicament destiné au traitement de maladies dans la pathogenèse desquelles l'élastase et/ou des métalloprotéases sont concernées.

2. Utilisation suivant la revendication 1, **caractérisée en ce que** le médicament sert au traitement de maladies du groupe : arthrite rhumatoïde, parodontite, plaques artériosclérotiques, ostéoporose, arthrose, ulcération de la cornée et réactions de la peau aux rayonnement ultraviolets.

3. Utilisation suivant la revendication 1, **caractérisée en ce que** le médicament sert au traitement de maladies du groupe : arthrite rhumatoïde, parodontite, plaques artériosclérotiques, ostéoporose, arthrose.

4. Utilisation du 4-(2-napht-2-yl-vinyl)-1,2-benzènediol sous forme des bases ou de sels acceptables du point de vue pharmaceutique pour la préparation d'un médicament destiné au traitement de maladies dans la pathogenèse desquelles l'élastase et/ou des métalloprotéases sont concernées.

5. Utilisation suivant la revendication 4, **caractérisée en ce que** le médicament sert au traitement de maladies du groupe : arthrite rhumatoïde, parodontite, ostéoporose, plaques artériosclérotiques et arthrose.
